# EUROPÄISCHE PATENTSCHRIFT

(11) **EP 3 330 683 B1**
(45) Veröffentlichungstag und Bekanntmachung des Hinweises auf die Patenterteilung: **08.01.2020**
(21) Anmeldenummer: 17210443.2
(22) Anmeldetag: 17.06.2015
(51) Int. Cl.: G01J 1/42, A61C 13/15

(54) **LICHTHÄRTGERÄT**
LIGHT HARDENING DEVICE
APPAREIL DE DURCISSEMENT À LA LUMIÈRE

(43) Veröffentlichungstag der Anmeldung: 06.06.2018
(62) Teilanmeldung aus: 15172551.2
(73) Patentinhaber: Ivoclar Vivadent AG, 9494 Schaan (LI)
(72) Erfinder: Senn, Bruno, 9056 Gais (CH); Tommasini, Dario, 7302 Mastrils (CH)
(74) Vertreter: Baronetzky, Klaus

(56) Entgegenhaltungen:
- EP-A1- 1 236 444
- DE-A1-102014 224 026
- JP-B2- 2 992 175
- US-B1- 6 485 301

## Beschreibung

Die Erfindung betrifft ein Lichthärtgerät, gemäß dem Oberbegriff von Anspruch 1.

Es ist seit langem bekannt, dass für die Gewährleistung einer zuverlässigen Polymerisation oder Härtung von Dentalmaterialien wesentlich ist, dass eine exakt vorgegebene Lichtmenge zugeführt werden muss. Die Soll-Lichtmenge hängt von vielen Faktoren ab, beispielsweise von der Form und der Schichtstärke sowie der Masse der zu härtenden Dentalrestauration, dem Photoinitiator, der die Polymerisation triggert, also auslöst, dem emittierten Wellenlängenbereich des Lichthärtgeräts an dessen Lichtleitstab, indirekt aber auch beispielweise von der Lichtquelle selbst, die eine Degradation erfahren kann, oder auch der Qualität des Lichtleitstabs, der beispielsweise auch gebrochen sein kann - ohne dass der Benutzer von etwaigen Rissen Kenntniss hat.

Es ist andererseits seit langem bekannt, dass man die Lichtquelle kalibrieren sollte, um die Lichtabgabe zu vergleichmäßigen. Als eine der zahlreichen Beispiele hierfür sei auf die DE 196 36 266 A1 verwiesen, gemäß welcher die dortige Lichtquelle von einem Detektor zur Messung der Lichtintensität in der Vorrichtung kontrolliert werden soll (vergleiche Spalte 3, Zeile 48 ff).

Nachteilig hierbei ist, dass der Lichtleitstab nicht in die Kalibration einbezogen werden kann.

Es sind aber auch bereits insofern verbesserte Lösungen bekannt geworden, die den Lichtleitstab einbeziehen. Hierzu sei auf die DE 10 2007 052 643 A1 verwiesen, die einen Lichtsensor an der Basisstation aufweist. Das Handstück muss bei dieser Lösung mit dem Ende des Lichtleitstabs auf die Sensorfläche gehalten werden, und es soll so die Lichtemission gemessen werden, insbesondere auch die Lichtintensität.

Wenn mehrere - gleichartige - Lichthärtgeräte verwendet werden, besteht die Möglichkeit, dass Basisstation und Handstück vom Zahnarzt nicht richtig zugeordnet werden, und die Messung der abgegebenen Lichtintensität sich dann auf das falsche Lichthärtgerät bezieht. Dies gilt im besonderen Maße, wenn die Übertragung der Kalibrierungsinformation lediglich dann erfolgt, wenn das Lichthärtgerät eingesteckt ist, wie es bei der Lösung gemäß der DE 10 2007 052 643 A1 der Fall ist, bei der die gemessene Lichtleistung dann in der Basisstation zwischengespeichert werden muss.

Ferner wäre die noch zuverlässigere Erkennung eines Mikrobruchs in dem Lichtleitstab wünschenswert.

Die EP 1 236 444 A1 offenbart eine Einrichtung zur Lichthärtung von Kunststoffen mit einer Lichtleitvorrichtung, welche neben Lichtleitfasern für die Hauptlichquelle auch einen zusätzlichen Lichtleiter für vom bestrahlten Objekt zurückgeworfene Lichtstrahlung. Durch ein Einschalten der Lichtquelle mit verminderter Leistung und die Erfassung der reflektierten Strahlung mittels eines im Handstück angeordneten Sensors soll eine automatische Einschaltung der Maximalleistung der Lichtquelle bei Überschreitung eines Schwellwertes der reflektierten Strahlung ermöglicht werden. Weiterhin ist dem Ende der Lichtleitervorrichtung ein aufsetzbarer Messtubus mit einer total reflektierenden Stirnfläche zugeordnet, mit dem das optische System auf seine Leistungsfähigkeit überprüft werden können soll. Da jedoch die Lichtstrahlung zum Objekt, also zum Messtubus, und die zurückgeworfene Strahlung durch unterschiedliche Lichtleitfasern geführt werden, ist eine sichere Erkennung von Faserbrüchen in der Lichtleitvorrichtung nicht möglich.

Daher liegt der Erfindung die Aufgabe zugrunde, ein Lichthärtgerät für das Härten von Dentalmaterialien gemäß dem Oberbegriff von Anspruch 1 zu schaffen, das hinsichtlich der Erkennungsleistung und der Zuverlässigkeit noch weiter verbessert ist, wobei die Kosten für die Realisierung sich höchstens im Rahmen der bereits bekannten Lösungen bewegen sollen.

Diese Aufgabe wird erfindungsgemäß durch Anspruch 1 gelöst. Vorteilhafte Weiterbildungen ergeben sich aus den Unteransprüchen.

Überraschend lässt sich mit der erfindungsgemäßen Lösung, einen preisgünstigen Spiegel zu verwenden, dessen reflektiertes Licht erneut durch den Lichtleitstab hindurchgeführt wird, die Erkennbarkeit von Mikrorissen deutlich verbessern. Die Mikrorisse streuen regelmäßig das eingeleitete Licht und erhöhen insofern die Dämpfung durch den Lichtleitstab; durch die erfindungsgemäße Verdopplung der Dämpfung gegenüber dem ordnungsgemäßen Betrieb des Lichtleitstabs bei der Kalibration lässt sich die Erkennungsrate deutlich verbessem.

Dadurch, dass die Steuervorrichtung dem Handstück unmittelbar zugeordnet ist, ist eine Verwechslung bzw. eine Fehlzuordnung der ermittelten Daten unmöglich; überraschend lässt sich daher dieses im Stand der Technik vorhandene Problem mit ausgesprochen einfachen Maßnahmen und dementsprechend preisgünstig sicher vermeiden.

Erfindungsgemäß lässt sich nun von dem Lichteinfall auf den Sensor, der sich in dem Handstück befindet, ein Kalibrierprogramm auslösen. Hierfür ist es bevorzugt, dass ein Schwellwert eingerichtet wird, damit nicht von der Reflexion an beispielsweise einer Zahnoberfläche bereits das Kalibrierprogramm gestartet wird.

Alternativ ist es auch möglich, das Kalibrierprogramm zu starten, indem ein Mikroschalter betätigt wird, der dann ausgelöst wird, wenn das Ende des Lichtleitstabs in einer vorgegebenen Position bezogen auf den erfindungsgemäßen Spiegel ausgerichtet ist. Eine dritte Möglichkeit besteht darin, das Kalibrierprogramm dann auszulösen, wenn der Benutzer dies wünscht.

Erfindungsgemäß günstig ist es, dass der Spiegel an oder zumindest in der Nähe des Handstücks angeordnet sein sollte. Die Anordnung an dem Handstück lässt sich beispielsweise durch einen Spiegelaufsatz am vorderen Ende des Lichtleitstabs realisieren. Dieser ist im "Normalbetrieb" zurückgeschwenkt und legt sich bevorzugt vergleichsweise eng an den Lichtleitstab an. Im Kalibrierbetrieb ist er nach vorne geschwenkt und erstreckt sich mit der Spiegelfläche parallel zum Auslassende des Lichtleitstabs, kurz vor diesem, so dass die emittierte Strahlung mit dem Reflektionswirkungsgrad des Spiegels im Wesentlichen vollständig reflektiert wird.

Es ist auch möglich, zusätzlich zu der Schwenkbarkeit den Spiegelaufsatz des Lichtleitstabs mit einer Schiebehülse zu versehen, so dass die Aufsteckhülse zur Wurzel des Lichtleitstabs zurückgeschoben wird und der Lichtleitstab dann an dem in den Mund einzuführenden Bereich frei von Zusatzelementen ist.

Günstig ist in diesem Zusammenhang, dass der Aufsatz nicht die Autoklavierbarkeit des steckbaren Lichtleitstabs behindert.

In einem alternativen Ausführungsbeispiel, das erläuterungshalber beigefügt ist, kann der Spiegel an der Basisstation angebracht sein. Dort ist es bevorzugt, dass ebenso wie bei der Aufsteckhülse eine Ausrichthilfe realisiert ist, die gewährleistet, dass sich der Spiegel im Kalibriermodus exakt senkrecht zu der Lichtaustrittsfläche des Lichtleitstabs erstreckt. Die Ausrichthilfe kann beispielsweise durch eine Hülse realisiert sein, in die der Lichtleitstab mit seinem vorderen Ende einsteckbar ist und die dieses in einem Winkel von 90° also senkrecht zu dem Spiegel hält. Auch diese Ausgestaltung gewährleistet, dass stets die gleichen Kalibrierbedingungen im Kalibriermodus vorliegen.

Der Sensor ist in dem Handstück so angeordnet, dass er die reflektierte Strahlung, die den Lichtleitstab erneut durchtritt, erfasst. Bevorzugt ist er optisch entkoppelt von der Lichtquelle angebracht, beispielsweise im Strahlengang dieser hinter dieser und er erhält über optische Koppelelemente die reflektierte Strahlung des Spiegels, nachdem sie den Lichtleitstab durchtreten hat.

In vorteilhafter Ausgestaltung ist es vorgesehen, dass dem Benutzer des Lichthärtgeräts die Qualität und aktuelle Leistung des Lichthärtgeräts des Kalibriermodus in geeigneter Weise angezeigt wird, sei es durch Farbanzeigen wie rot, gelb, grün oder als numerischer Wert unter Angabe der Lichtleistung. Der Benutzer kann so frühzeitig eine Degradation eines Lichthärtgeräts feststellen und beispielsweise dafür sorgen, dass Verschmutzungen die Lichtleistungen nicht beeinträchtigen.

Es ist auch möglich, mit einer entsprechenden Anzeige den Benutzer zur Reinigung oder zum Austausch des Lichtleiters aufzufordern.

Steckbare Lichtleiter sind im Grunde aufgrund der Autoklavierbarkeit bevorzugt. Sie bergen jedoch die Gefahr, dass, wenn der Lichtleitstab versehentlich nach der Reinigung nicht erneut eingesteckt wird, das Linsensystem des Lichthärtgeräts verschmutzt, beispielsweise verstaubt. Dies führt ebenfalls zur Degradation der Lichtleistung, wobei es aber möglich ist, dass der Benutzer gemäß Betriebsanleitung des Lichthärtgeräts auch hier selbst eine Reinigung vornimmt, beispielsweise mit einem weichen Pinsel oder ähnlichem die Optik reinigt.

Der Lichtleitstab selbst kann insbesondere an seinem vorderen Ende, das häufig Kontakt mit dem Mund des Patienten hat, Ablagerungen tragen, die durch Autoklavieren nicht oder nicht vollständig beseitigt werden. Hier kann dem Benutzer eine Reinigung dieses vorderen Endes per Anzeige empfohlen werden, so dass ein vorzeitiger Austausch des im übrigen noch funktionsfähigen Lichtleitstabs vermieden werden kann.

Andererseits können Lichtleitstäbe Mikrorisse aufweisen, also Risse, die von außen nicht ohne weiteres zu erkennen sind - wenn nicht die typischerweise schwarze Kunststoffschutzhülle entfernt wird. Der Lichtleitstab ist in diesem Zustand typischerweise nicht vollständig gebrochen, sondern weist lediglich Risse auf, die beispielsweise durch eine unsaubere Handhabung, durch Fallenlassen oder dergleichen hervorgerufen worden sind.

Diese Mikrorisse führen zur Erhöhung der Dämpfung der Durchlässigkeit des Lichtleitstabs. Bei einem typischen Riss ist die Lichtleistung im Betrieb dann beispielsweise auf den Faktor 0,8 abgesenkt. Erfindungsgemäß ist die Signifikanz dieser Verschlechterung im Kalibriermodus jedoch wesentlich erhöht; die reflektierte Strahlung wird erneut um den Faktor 0,8 reduziert, so dass sich insgesamt eine Reduktion um 0,8 mal 0,8, also 0,64, ergibt, die wesentlich besser von dem Sensor und der zugehörigen Steuereinrichtung des Lichthärtgeräts erkennbar ist.

Erfindungsgemäß ist es vorgesehen, im Kalibriermodus unkontinuierliches Licht von der Lichtquelle austrahlen zu lassen, beispielsweise sinusförmiges oder gepulstes Licht. Dies ermöglicht die Unterscheidung zwischen Umgebungslicht und dem emittierten Licht der Lichtquelle. Hierbei wird von der Steuervorrichtung dann kurzerhand die Amplitude des empfangenen Ausgangssignals des Sensors ausgewertet und als Nutzlicht für die Kalibrierung verwendet.

Innerhalb des Regelbereichs lässt sich im Kalibriermodus auch eine Selbstnachkalibration realisieren; beispielsweise kann eine Erhöhung oder Erniedrigung der Lichtleistung um plus/minus 20% vorgegeben sein.

Erfindungsgemäß günstig ist es auch, wenn das Handstück des Lichthärtgeräts die Entnahme des Lichtleiters automatisch erkennt und dann umgehend den Kalibriermodus anfordert bzw. beim nächsten Einschalten hierzu auffordert.

Dies ermöglicht die automatische Kompensation der Exemplarstreuung von Lichtleitstäben beim Tausch der Lichtleitstäbe. Zudem wird der Tatsache damit Rechnung getragen, dass beim Reinigen des Lichtleitstabs die Durchlässigkeit meist verbessert ist, so dass insofern eine Kalibration empfehlenswert ist.

Bevorzugt sind der oder die Sensoren etwas außerhalb der Zentralachse des rückwärtigen Endes des Lichtleitstabs angeordnet. Sie erfassen damit bevorzugt das am Außenumfang des Lichtleitstabs übertragene reflektierte Licht, während die Lichtquelle bevorzugt den zentralen Bereich des Lichtleitstabs beaufschlagt.

Weitere Vorteile, Einzelheiten und Merkmale ergeben sich aus der nachfolgenden Beschreibung eines Ausführungsbeispiels der Erfindung anhand der Zeichnungen.

Es zeigen:
- Fig. 1: eine schematische Ansicht einer Ausführungsform eines Lichthärtgeräts, das erläuterungshalber beigefügt ist, in perspektivischer Darstellung;
- Fig. 2: die Draufsicht auf die Ausführungsform gemäß Fig. 1; und
- Fig. 3: eine leicht schematisierte Ansicht einer erfindungsgemäßen Ausführungsform eines erfindungsgemäßen Lichthärtgeräts

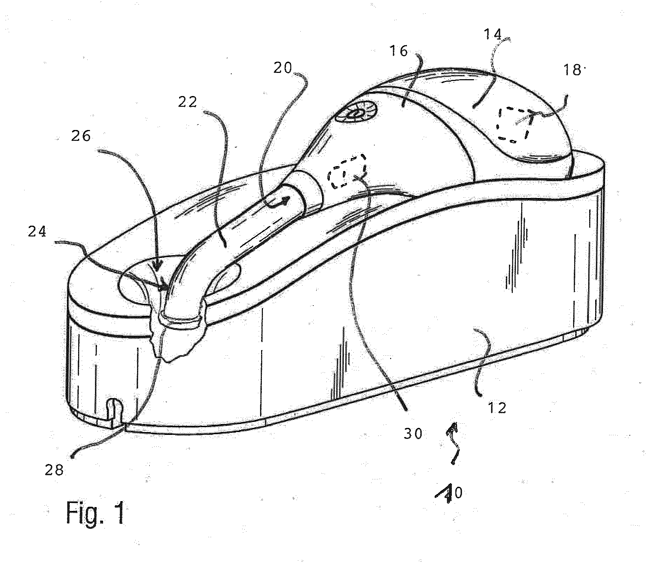

Das in Fig. 1 dargestellte Lichthärtgerät 10 weist eine Basisstation 12 und ein Handstück 14 auf, die in an sich bekannter Weise voneinander trennbar sind. Die Basisstation 12 bietet in dem dargestellten Ausführungsbeispiel eine räumlich fixierte Auflage bzw. Einlage für das Handstück 14, und zudem die Möglichkeit, den Akkumulator des Handstücks 14 automatisch aufzuladen.

Ferner ist eine bevorzugt drahtlose Datenübertragung zwischen Handstück 14 und Basisstation 12 vorgesehen. Diese dient beispielsweise der Anpassung des Ladestroms an die Erfordernisse. Eine bidirektionale Kommunikation ist optional auch möglich, beispielsweise, wenn Daten über den Betrieb des Handstücks in der Zahnarztpraxis, die beispielsweise mehrere erfindungsgemäße Lichthärtgeräte haben kann, an eine Zentrale übetragen werden sollen.

Das Handstück 14 weist ein Gehäuse 16 auf, das eine schematisch dargestellte Steuervorrichtung 18 aufnimmt. Das Gehäuse 16 weist an seinem vorderen Ende eine Aufnahme 20 für einen Lichtleitstab 22 auf. In dem dargestellten Ausführungsbeispiel ist der Lichtleitstab 22 austauschbar und rastet in der Aufnahme 20 ein; in einer anderen Ausführungsform ist er fest mit dem Gehäuse 16 verbunden.

In an sich bekannter Weise ist der Lichtleitstab 22 abgekröpft, und zwar vor seinem vorderen Ende 24. Dieses liegt in der dargestellten Ruheposition in einer Vertiefung 26 der Basisstation 12, wobei das Lichtaustrittsende des Lichtleitstabs 22 in die Vertiefung 26 hineinragt.

Die Basisstation 12 weist unten in der Vertiefung 26 einen Spiegel 28 auf, der genau zu dem vorderen Ende 24 des Lichtleitstabs 22 passt und gegenüber diesem exakt ausgerichtet ist, wenn das Handstück 14 in der Ruheposition in der Basisstation 12 liegt.

Der Spiegel ist leicht schräg gegenüber der Horizontalen ausgerichtet, entsprechend der Lichtaustrittsfläche des Lichtleitstabs und weist im sauberen Zustand einen Reflexionswert von mehr als 95% auf. Hierdurch wird das aus dem Lichtleitstab 22 austretende Licht nahezu vollständig reflektiert und in den Lichtleitstab 22 zurückgeworfen.

Dem hinteren Ende des Lichtleitstabs 22 und der Aufnahme 20 benachbart ist ein Sensor 30 in dem Handstück 14 vorgesehen, der das von dem Spiegel 28 zurückgeworfene und erneut durch den Lichtleitstab 22 hindurchgeführte Licht erfasst und dessen Ausgangssignal der Steuervorrichtung 18 zugeleitet wird.

Die Steuervorrichtung 18 weist nun ein spezielles Kalibrierprogramm auf, das bei einem bestimmten Lichteinfall auf den Sensor 30 bzw. alternativ auch bei mechanischer Aktivierung durch den benutzenden Zahnarzt ein Kalibrierprogramm auslöst. Das Kalibrierprogramm regelt eine Nenn-Lichtleistung der Lichtquelle des Handstücks 14, die ebenfalls der Aufnahme 20 benachbart ist, auf einen Nennwert, so dass die Lichtabgabe im Betrieb stets in gleicher Weise erfolgt.

In einer weiteren alternativen Ausgestaltung ist es vorgesehen, dass das Kalibrierprogramm jedes mal automatisch gestartet wird, wenn das Handstück 14 in die Basisstation 12 eingelegt wird. Die Anpassung der Lichtleistung an den Sollwert erfolgt dann automatisch innerhalb der Regelreserve.

Wenn die Regelreserve der Lichtleistung nahezu aufgebraucht ist, erfolgt bevorzugt eine Nachricht an den Zahnarzt oder sonstigen Nutzer, mit welcher dieser veranlasst werden sollte, den Lichtleitstab zu reinigen oder zu ersetzen.

Es versteht sich, dass die erläuterungshalber beigefügte Lösung eines Lichthärtgeräts mit integriertem Spiegel nicht auf stiftförmige Handstücke beschränkt ist; vielmehr können in gleicher Weise auf pistolenförmige Lichthärtgeräte, wie sie weit verbreitet sind, entsprechend erfindungsgemäß bestückt sein.

Der Spiegel 28 muss auch nicht an der Stelle angeordnet sein, an welcher das Handstück sich in der Ruhestellung befindet; dies ist jedoch bevorzugt. Alternativ kann beispielsweise eine Vertiefung 26 an der Seite der Basisstation 12 vorgesehen sein, die mit dem Spiegel 28 bestückt ist und in welche das vordere Ende 24 des Lichtleitstabs 22 einführbar ist.

Diese Vertiefung 26 bietet dann eine Ausrichthilfe für die Ausrichtung des Lichthärtgeräts gegenüber dem Spiegel 28, ebenso wie die Vertiefung 26 gemäß Fig. 1 insofern eine Ausrichthilfe bietet.

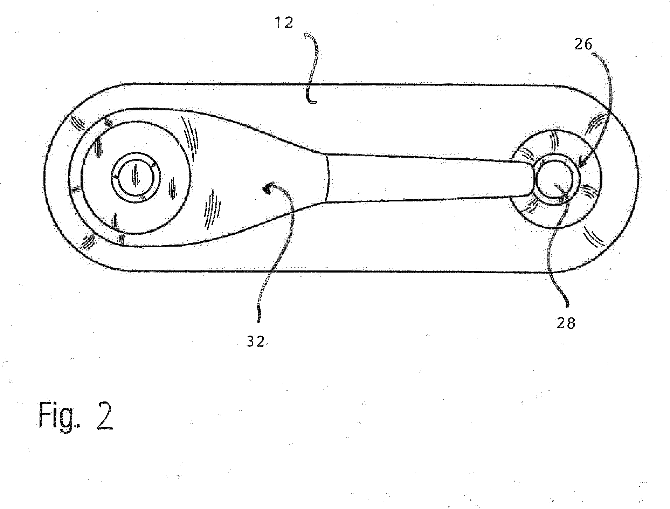

Aus Fig. 2 ist die Basisstation 12 in der Draufsicht ersichtlich. Die Basisstation 12 weist praktisch eine Art Ladeschale 32 für das Handstück 14 auf. Es ist der freiliegende Spiegel 28 ersichtlich, der am Böden der Vertiefung 26 angebracht ist. Da die Vertiefung 26 abgerundet ist, lässt sich der Spiegel relativ leicht sauber halten, und die räumliche Fixierung, also die Ausrichthilfe erfolgt zwischen der Außenkontur des Handstücks 14 und der Ladeschale 32 der Basisstation 12.

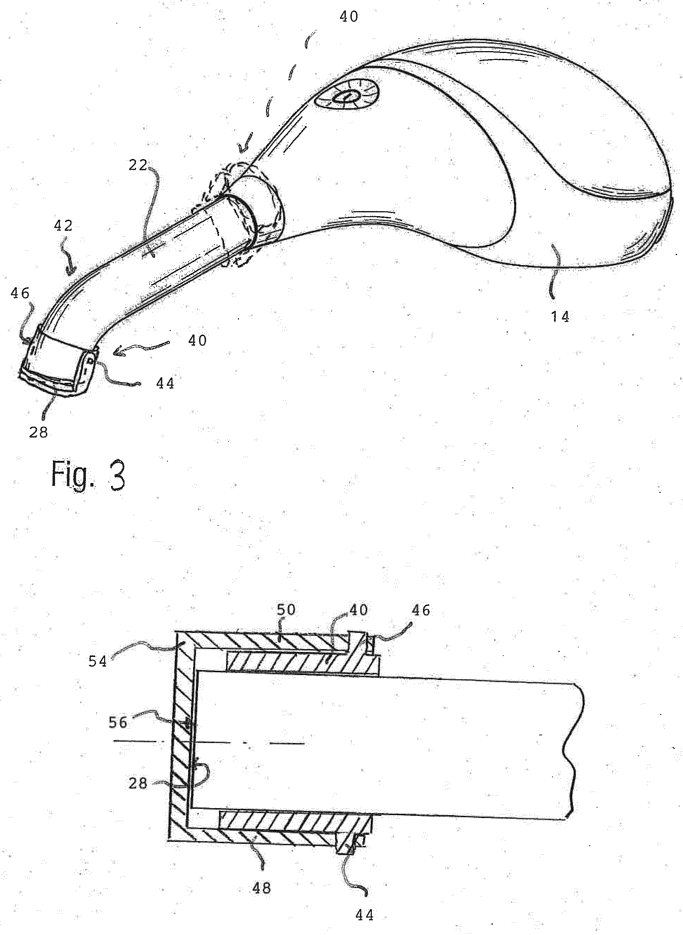

Aus Fig. 3 ist eine Ausgestaltung des erfindungsgemäßen Lichthärtgeräts ersichtlich. Bei dieser Ausführungsform ist der Spiegel 28 am Handstück 14 selbst angebracht. Es ist eine Schiebehülse 40 vorgesehen, die mit ihrem Innendurchmesser zum Außenumfang des Lichtleitstabs 22 passt. Die Schiebehülse 40 besteht aus einem Elastomer und haftet unter einer gewissen Vorspannung auf dem Lichtleitstab. Aufgrund ihrer Elastizität lässt sie sich auch über die Krümmung 42 des Lichtleitstabs hinwegschieben.

Weiter unten in Fig. 3 ist der Aufbau im Schnitt dargestellt.

Die Schiebehülse 40 trägt seitlich zwei Lagerzapfen 44 und 46. Die Lagerzapfen werden von Halterarmen 48 und 50 einer Spiegellagerung 54 übergriffen und sind an diesen schwenkbar. Die Spiegellagerung 54 trägt den Spiegel 28, dessen Durchmesser etwas größer als der Durchmesser der Lichtaustrittsfläche 56 des Lichtleitstabs 22 ist. Diese Elemente bieten insofern einen Spiegelaufsatz.

Die Spiegellagerung 54 kann durch Schwenken um die Zapfen 44 und 46 in eine seitliche Position, aber auch die in der Figur dargestellte Kalibrierposition verschwenkt werden. In dieser liegt der Spiegel 28 an der Lichtaustrittsfläche 56 an, die insofern eine Ausrichtshilfe bildet.

Im Betrieb wird nun die Schiebehülse 40 zunächst soweit nach Vorne geschoben, dass ein Verschwenken der Spiegellagerung 54 möglich ist. Dann wird sie erneut zurückgeschoben, so dass der Spiegel 28 auf der Lichtaustrittsfläche 56 aufliegt. In diesem Zu- stand wird der Kalibriermodus eingeschaltet und die Kalibrierung wird in der oben beschriebenen Weise vorgenommen.

Nachdem die Kalibrierung erfolgt ist, wird die Schiebehülse 40 entweder kurzerhand abgezogen, oder sie wird in die in Fig. 3 gestrichelt dargestellte rückwärtige Position geschoben, natürlich nachdem die Spiegellagerung 54 in die Seitwärtsposition verschwenkt ist.

In der rückwärtigen Position der Spiegellagerung 54 und der Schiebehülse 40 liegt der Lichtleitstab 22 im übrigen frei, und es ergibt sich keine Behinderung bei der Lichthärtung im Mund des Patienten.

In einer bevorzugten Ausführungsform ist die Spiegellagerung mit an sich bekannten Blendschutzklappen kombiniert, die bei nach außen verschwenkter Spiegellagerung gleichzeitig einen Blendschutz ermöglichen.

In einer weiteren Ausführungsform, die erläuterungshalber beigefügt ist, ist das Spiegelement aus Klebefolie ausgebildet, die auf die Lichtaustrittsfläche 56 aufgeklebt wird und nach Gebrauch verworfen wird.

In einer weiteren Ausführungsform ist es vorgesehen, die Lichtquelle in dem Handstück an das vordere Ende eines Stabes zu verlagern, der dann nicht als Lichtleitstab, sondern als Spannungsversorgungs- und Kühlstab ausgebildet ist. Auch der zugehörige Sensor 30 ist dann an dem vorderen Ende des Handstücks angeordnet, und zwar neben der Lichtquelle, bevorzugt radial außerhalb dieser.

## Patentansprüche

1. Lichthärtgerät für das Härten von Dentalmaterialien, mit einem Handstück (14) und einer Basisstation (12), wobei das Handstück (14) eine Lichtquelle, einen Lichtleitstab (22), der insbesondere austauschbar und lösbar an dem Handstück (14) befestigt ist, sowie mindestens einen Sensor (30) aufweist, der sich im Handstück (14) befindet, wobei das Lichthärtgerät (10) weiter einen Spiegel (28), der an dem Handstück (14) des Lichthärtgeräts (10) vorgesehen ist, mit vorgegebenem Reflexionsparameter mit einem Reflexionswert von mehr als 90% und eine Steuervorrichtung (18) mit mindestens einem Härtprogramm für das Polymerisieren des Dentalmaterials sowie ein Kalibrierprogramm aufweist, wobei das Kalibrierprogramm automatisch, wenn das Lichthärtgerät (10) mit dem lichtaustrittsseitigen Ende (24) des Lichtleitstabs (22) auf den Spiegel (28) gerichtet ist, oder per Benutzereingriff auslösbar ist, **dadurch gekennzeichnet,**
**dass** der Spiegel (28) als Messspiegel an einem Blendschutz schwenkbar angebracht ist, der am Lichtaustrittsende des Lichtleitstabs (22) befestigt ist.

2. Lichthärtgerät nach Anspruch 1, **dadurch gekennzeichnet, dass** ein Spiegelaufsatz am lichtaustrittsseitigen Ende (24) des Lichtleitstabs (22) vorgesehen ist und eine Schiebehülse (40) sowie eine Spiegellagerung (54) aufweist, die den Spiegel (28) trägt, wobei die Spiegellagerung (54) mit Blendschutzklappen kombiniert ist, die bei nach außen verschwenkter Spiegellagerung (54) gleichzeitig einen Blendschutz ermöglichen.

3. Lichthärtgerät nach Anspruch 1, **dadurch gekennzeichnet, dass** der Sensor (30) und die Lichtquelle einem Lichteintrittsende des Lichtleitstabs (22) benachbart angeordnet sind.

4. Lichthärtgerät nach einem der Ansprüche 1 oder 3, **dadurch gekennzeichnet, dass** das Kalibrierprogramm die Nenn-Lichtleistung der Lichtquelle basierend auf dem Messergebnis des Sensors (30) einstellt.

5. Lichthärtgerät nach einem der Ansprüche 1, 3 oder 4, **dadurch gekennzeichnet, dass** das Handstück (14) einen Präsenssensor für den Lichtleitstab (22) aufweist, mit welchem feststellbar ist, ob ein Lichtleitstab (22) an dem Handstück (14) eingesteckt oder nicht eingesteckt, und dass die Steuervorrichtung (18) automatisch das Eigenkalibrationsprogramm durchführt oder zu diesem auffordert, wenn der Präsenssensor den Wechsel des Lichtleitstabs (22) an die Steuervorrichtung (18) gemeldet hat.

6. Lichthärtgerät nach einem der Ansprüche 1, 3 bis 5, **dadurch gekennzeichnet, dass** die Steuervorrichtung (18) an eine Anzeigevorrichtung angeschlossen ist, die den Betriebszustand des Lichthärtgeräts (10) anzeigt, insbesondere die Durchführung des Kalibrationsprogramms und den Benutzer auf die Sperre der normalen Betriebsfunktion des Lichthärtgeräts (10) während des Kalibration hinweist.

7. Lichthärtgerät nach Anspruch 6, **dadurch gekennzeichnet, dass** die Anzeigevorrichtung die von dem optischen Sensor (30) gemessene und für die Kalibrierung verwendete Lichtleistung als numerischen Wert angibt.

8. Lichthärtgerät nach einem der Ansprüche 1, 3 bis 7, **dadurch gekennzeichnet, dass** das Eigenkalibrationsprogramm der Steuervorrichtung (18) den Lichthärtezyklus bei schwindender und dementsprechend geringer gemessener Leistung der Lichtquellen proportional um bis zu 20% verlängert und dass die Belichtungszeit automatisch verlängert wird, also zum Beispiel von 5 Sekunden auf 6 Sekunden.

## Claims

1. A light hardening device for hardening of dental materials, comprising a handpiece (14) and a base station (12), the handpiece (14) having a light source, a light guide rod (22), which especially is exchangeably and detachably fastened to the handpiece (14), and at least one sensor (30), which is located In the handpiece (14), the light hardening device (10) further comprising a mirror (28), which is provided on the handpiece (14) of the light hardening device (10), having a predetermined reflection parameter with a reflection value of more than 90%, and a control device (18) with at least one hardening program for polymerizing the dental material and a calibration program, the calibration program being automatically initiated or being able to be initiated by user Intervention when the light-hardening device (10), with the light-emitting end (24) of the light guide rod (22), is directed onto the mirror (28), **characterized in that** that the mirror (28) is pivotably mounted as a measuring mirror on a glare shield which glare shield is fastened to the light-emitting end of the light guide rod (22).

2. The light hardening device according to claim 1, **characterized in that** a mirror attachment Is provided at the light-emitting end (24) of the light guide rod (22) and comprises a sliding sleeve (40) and a mirror bearing (54) which supports the mirror (28), wherein the mirror bearing (54) is combined with anti-glare flaps, which simultaneously provide glare protection when the mirror bearing (54) is swiveled outwards.

3. The light hardening device according to claim 1, **characterized in that** the sensor (30) and the light source are arranged adjacent to a light entry end of the light guide rod (22).

4. The light hardening device according to any of claims 1 or 3, **characterized in that** the calibration program sets the nominal light output of the light source based on the measurement result of the sensor (30).

5. The light hardening device according to any of claims 1, 3 or 4, **characterized in that** the handpiece (14) has a presence sensor for the light guide rod (22) with which it may be determined whether a light guide rod (22) Is plugged into the handpiece (14) or not, and **in that** the control device (18) automatically executes the self-calibration program or requesting it when the presence sensor has reported the change in the light guide rod (22) to the control device (18).

6. The light hardening device according to any of claims 1,3 to 5, **characterized In that** the control device (18) is connected to a display device Indicating the operating state of the light hardening device (10), especially execution of the calibration program, and Indicating regular operating function suspension of the light hardening device (10) during calibration to the user.

7. The light hardening device according to claim 6, **characterized in that** the display device indicates the light power measured by the optical sensor (30) and used for calibration as a numerical value.

8. The light-hardening device according to any of claims 1, 3 to 7, **characterized in that** the self-calibration program of the control device (18) proportionally extends the light-hardening cycle by up to 20% when the power of the light sources diminishes and will correspondingly be measured as being lower, and **In that** the exposure time is automatically extended, for example from 5 seconds to 6 seconds.

## Revendications

1. Dispositif de photopolymérisation pour photopolymériser des matériaux dentaires, comprenant une pièce à main (14) et une station de base (12), où la pièce à main (14) présente une source de lumière, une tige de guidage de lumière (22), qui est en particulier fixée de manière Interchangeable et amovible à la pièce à main (14), et au moins un capteur (30) qui est situé dans la pièce à main (14), où le dispositif de photopolymérisation (10) présente en outre un miroir (28) prévu sur la pièce à main (14) du dispositif de photopolymérisation (10) et ayant un paramètre de réflexion prédéterminé avec une valeur de réflexion supérieure à 90% et un dispositif de commande (18) ayant au moins un programme de polymérisation pour la polymérisation du matériau dentaire et un programme de calibration, où le programme de calibration peut être déclenché de manière automatique lorsque le dispositif de photopolymérisation (10) est dirigé avec l 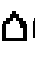 extrémité du côté de l'émergence de lumière (24) de la tige de guidage de lumière (22) vers le miroir (28) ou par une intervention de l'utilisateur, **caractérisé en ce que** le miroir (28) est monté de manière pivotante en tant que miroir de mesure sur une protection anti-éblouissement qui est fixée à l'extrémité de l'émergence de la lumière de la tige de guidage de lumière (22).

2. Dispositif de photopolymérisation selon la revendication 1, **caractérisé en ce qu'**une fixation de miroir est prévue à l'extrémité du côté de l'émergence de la lumière (24) de la tige de guidage de lumière (22) et présente un manchon coulissant (40) ainsi qu'un support d'un miroir (54) qui porte le miroir (28), où le support de miroir (54) est combiné avec des volets anti-éblouissement qui, lorsque le palier de miroir (54) est pivoté vers le extérieur, permettent simultanément une protection anti-éblouissement.

3. Dispositif de photopolymérisation selon la revendication 1, **caractérisé en ce que** le capteur (30) et la source lumineuse sont disposés à proximité d'une extrémité d'entrée de lumière de la tige de guidage de lumière (22).

4. Dispositif de photopolymérisation selon l'une quelconque des revendications 1 ou 3, **caractérisé en ce que** le programme de calibrage règle la puissance lumineuse nominale de la source lumineuse en fonction du résultat de mesure du capteur (30).

5. Dispositif de photopolymérisation selon l'une quelconque des revendications 1, 3 ou 4, **caractérisé en ce que** la pièce à main (14) comprend un capteur de présence pour la tige de guidage de lumière (22), avec lequel peut être détecté si une tige de guidage de lumière (22) a été inséré ou non dans la pièce à main (14), et **en ce que** le dispositif de commande (18) exécute automatiquement le programme d'auto-calibrage ou invite à l'exécuter lorsque le détecteur de présence a signalé au dispositif de commande (18) le changement de la tige de guidage optique (22).

6. Dispositif de photopolymérisation selon l'une quelconque des revendications 1, 3 à 5, **caractérisé en ce que** le dispositif de commande (18) est relié à un dispositif d'affichage qui indique l'état de fonctionnement du dispositif de photopolymérisation (10), en particulier l'exécution du programme de calibrage, et signale à l'utilisateur la suspension du fonctionnement normal du dispositif (10) pendant le calibrage.

7. Dispositif de photopolymérisation selon la revendication 6, **caractérisé en ce que** le dispositif d'affichage affiche la puissance lumineuse mesurée par le capteur optique (30) et utilisée pour le calibrage comme valeur numérique.

8. Dispositif de photopolymérisation selon l'une des revendications 1, 3 à 7, **caractérisé en ce que** le programme d'auto-calibrage du dispositif de commande (18) prolonge proportionnellement le cycle de photopolymérisation jusqu'à 20 % lorsque la puissance des sources lumineuses diminue et par conséquent est mesurée plus faible, et que le temps d'exposition est automatiquement prolongé, par exemple de 5 secondes à 6 secondes.
